# EUROPEAN PATENT APPLICATION

(11) **EP 0 916 947 A1**
(43) Date of publication of application: **19.05.1999**
(21) Application number: 97203532.3
(22) Date of filing: 12.11.1997
(51) Int. Cl.: G01N 33/12, G01N 3/42

(54) **Method and device for measurement of tension or tenderness of animal tissue such as meat**

(71) Applicant: Stichting Instituut voor Dierhouderij en Diergezondheid (ID-DLO), 8219 PH Lelystad (NL)
(72) Inventor: Hopster, H., 8219 PH Lelystad (NL)
(74) Representative: Smulders, Theodorus A.H.J., Ir.

(57) **Abstract**

Method for measurement of tension or tenderness of animal tissue such as meat, using an instrument having at least one measurement means and reference means, whereby the measurement means is positioned in a first position, against part of a tissue to be measured, whereafter the measurement means and/or the reference means are moved relative to the tissue to a second position, thereby deflecting the tissue, after which the measurement means and/or the reference means are moved back in the direction of the first position, wherein during at least part of the path of travel of the measurement means the relation is established between at least two of the following parameters of the movement of the measurement means relative to the reference means:
time, displacement, speed, change in speed, acceleration,
change in acceleration, duration of movement, pressure,
change in pressure,
   which relation is used for assessment of said tension or tenderness.

## Description

The invention relates to a method for measurement of tension or tenderness of animal tissue such as meat. Such a method is known and used in the meat industry.

A major problem facing the modern meat industry is the difficulty of predicting the yield and quality of meat from an outward inspection of a carcass, although the judgements of an experienced meat buyer may be quite useful. From combining knowledge about the age or maturity of an animal at slaughter, about the sex, type or breed of animal and from overall shape, weight and fatness of the carcass, subjective predictions may be made about the anticipated tenderness, colour, marbling and water-holding capacity of meat, but such predictions are only reasonable in the realm of averages and probabilities.

In the meat industry there are two terms, PSE and DFD, describing specific conditions of meat quality. PSE stands for pale, soft and exudative. It is a long standing problem in pork and a growing concern in white poultry meats and may occur as a rarity in dark meats such as beef or lamb. DFD stands for dark, firm and dry and is the opposite condition of PSE. DFD is a minor problem in pork but is sometimes a major problem in beef. Meat in PSE or DFD, respectively, is pale or dark and scatters more or less light than normal because of a low or high pH, and it is soft or firm and exudative or dry because of fluid located between or within the muscle fibers. Both PSE and DFD meat are not valued by the final consumer who will cook the PSE meat much longer than needed in order to reduce the wateriness of the meat, with the end result of a tough piece of meat, whereas DFD meat is already tough to begin with and cannot be improved by cooking.

In more general terms, the main concerns of lean pork and beef lay with pH-related quality problems and with tenderness.

Tenderness is on the one hand highly valued by the final customer but is on the other hand a meat quality that is very hard to measure reliably. Identification of poultry with tender meat is a complex subjective valuation based on a number of tactile inputs. Indicative of tenderness, the skin is judged to be soft, pliable and smooth-textured, and the sternal cartilage is judged to be flexible. Intermediate levels of tenderness resulting from advancing age are identified by increasing skin coarseness and decreased flexibility of the sternal cartilage. With larger animals other subjective valuation methods to assess and predict tenderness are being practised. For instance, shaking hands with a pig is an old custom for butchers. After the carcass of a slaughtered animal has been dressed and is hanging from a gambrel, its forelimp projects outwards and shaking the forelimp in a fore and afterwards rocking motion may enable the butcher to detect the early onset of rigor mortis in a carcass. Later on, a same shaking motion gives information about setting of the fat, which makes the meat stiff enough to cut neatly. Eventually, shaking hands may convey information about softening of the meat as it is becoming more tender, succulent and tasty.

Another mistical skill, only learned by practical experience, is how to judge softness, and thus tenderness of cutlets. Pushing the tip of the index finger into the horizontal surface of a cut longissimus dorsi may reveal much about the softness and the resilience of the meat and its future fluid losses. Sometimes the meat springs back when the finger tip is removed, sometimes it comes back slowly, and sometimes a depression remains and fills with fluid. Similarly, pinching a beef steak tightly between thump and forefinger may convey an impression of its future eating characteristics.

Furthermore, a method is known using a device, commonly known as a Sybesma rigorometer which is pushed against the tissue to be measured, by manual force. The rigorometer comprises a pusher element spring loaded into a first, neutral position. The pusher element is surrounded by a support structure relative to which the pusher element can be moved longitudinal, in an inward direction against the spring bias, a head of the pusher element extending outside the support structure in said neutral position. The support structure is provided with a measurement scale, the pusher element with a needle movable along said scale upon movement of the pusher element relative to the support structure. During use the head of the pusher element is positioned on the tissue to be measured, after which the support structure is pushed into the direction of the tissue, until a reference plane surrounding the head of the pusher element is in contact with said tissue. During this movement the needle is moved relative to the scale. This relative movement of the needle is supposed to provide an indicator for the tension or tenderness of said tissue.

A major disadvantage of the use of this known method is that the movement of the needle relative to the scale not only depends on the resilience, that is tension or tenderness of said tissue but is also influenced by the exact direction of movement of the rigorometer, the speed of the movement thereof relative to the tissue, the accuracy of reading the scale and, most important, the accuracy and experience of the user. Furthermore this known method can only be used for one specific muscle in the ham-region. The value obtained is a subjective value, which means that the values of subsequently measured products can only be compared to each other when taken by the same user, with the same accuracy and under the same circumstances. Such measurements are not sufficient for use with for example continuous and automatic assessment of the tension or tenderness of products in for example production lines in a slaughter house or the like or with living animals, especially not when important decisions are to be made based on said values and the experience of the user.

Chances are, for example, that the scale is being read to soon, the tissue not having returned to its final shape, while the reshaping can be significantly influenced by the Sybersma rigorometer itself and thus on the assessment of said tension or tenderness. A further disadvantage of these known methods is that the values measured by different users will not be sufficiently comparable due to different level of experience of said users. Therefore, it is not possible to maintain the same standards of quality and procedures, based on measured tension or tenderness.

One object of the present invention is to provide for a method for measurement of tension or tenderness of animal tissue, wherein the drawbacks of the known methods are overcome, maintaining the advantages thereof.

A further object of the present invention is to provide for such method enabling easy comparison of different measurements of tension or tenderness of animal tissue, thus providing for a method for easy maintenance of proper quality standards.

A still further object of the present invention is to provide for a method for measurement of the tension or tenderness of animal tissue which is easy to perform and can be automated thus enabling automatic processing of the meat or animals measured, based on the assessed tension or tenderness.

To this aim a method according to the present invention is characterized by the features of claim 1.

Establishment of the relation between at least two of the said parameters of the movement of the measurement means relative to the reference means enables accurate determination of at least part of the path of travel of said measurement head, uninfluenced by the experience of the user and independent of outside influences. The said parameters can easily be measured and for example fed into a computer having an algorithm for determining said relation or even relations between two or more of said parameters. This at least one relation can then be translated into a value representing the tension or tenderness of the tissue measured. Since it will only be necessary to measure said parameters during part of the movement of the measurement means, which duration can easily be set beforehand, or be introduced during processing of the data, unclear parts of the path of travel can be left out of the evaluation, for example at the start of movement of the measurement head or at the end thereof. Thus the measurements can be far more accurate then by using the known methods.

In a preferred embodiment a method according to the present invention is characterized by the features of claim 2.

Comparison of the established relation to a standard set of relations for similar tissue with known tension or tenderness provides for an even more accurate quality evaluation, uninfluenced by experience of the users. With this method the same quality standard can be maintained during a long period of time and can be adjusted, for example based on data obtained from consumer panels providing for subjective information of consumer acceptance of said tissue, which can be related to the measured and evaluated tension or tenderness of said tissue.

In a preferred embodiment a method according to the present invention is characterized by the features of claim 6.

By moving the measurement means against the surface of said tissue, avoiding penetration thereof, damage of the surface is prevented, thus maintaining the quality of said tissue. Furthermore, such method can be used with living animals, preventing discomfort and pain to the animal. Furthermore, such method can be more hygienic.

In a first preferred embodiment, the invention relates to a method for measurement of tenderness of meat in a slaughter house, meat industry, butchery or the like, characterized by the features of claim 8.

The electronic means of a self learning type enable easy automation of the meat processing within said slaughter house or the like, based on the assessed tension or tenderness of said meat.

The present invention also relates to a method for measurement of the tension in an udder or the like of a live mammal, characterized by the features of claim 9.

The high level of milk production of dairy cows causes significant threats to their welfare. Due to selective breeding, improved feeding and management and the use of bovin somatotropine, the level of milk production in dairy cows continues to increase. However, milking procedures did hardly change. On most dairy farms, cows are milked two times a day with intervals, varying between 9-15 h. The larger the milking interval and the higher the individual milk yield of the cow, the more milk has to be stored in the udder.

Milk stored in the bovine udder can be devided into 2 fractions with respect to the availability for milking. The cisternal milk fraction is located in the cisternal cavities of the teats and glands and in the larger milk ducts. This milk can easily be removed from the udder by overriding the contractile force of the teat duct sphincter (flaccid volume). In contrast, a large proportion of the milk remains in the secretory tissue after synthesis and is fixed primarily by adhesive and capillary forces. Generally, there is a lag, of around 6 h during which relatively little milk accumulates in the cistern. Thereafter, an increasing proportion of milk is stored in the secretory tissue. However, between individual cows, large differences have been found in the ratio between cisternal and secretory tissue storage capacities. In the high yielding dairy cow at the end of the milking interval, the accumulation of milk approaches the maximum storage capacity of the udder with accompanying increase of its tension. Dependent on the magnitude of the tension, this may cause discomfort and pain to the cow, especially when lying, thus impairing the welfare of the dairy cow.

Another important aspect of udder tension is related to milk production. The synthesis of milk in the secretory tissue is inhibited by the disruption of tight junction integrity, induced by the increase of intra-mammary pressure. In order to maximize milk synthesis, it has to be prevented that the intra-mammary pressure reaches the inhibitory level.

Because there is considerable variability in bovine cistern proportion and storage capacity on the one hand and milk production level and stage of lactation on the other, equally (long) milk intervals may impair the welfare of individual dairy cows differently.

A method according to the present invention provides for a non-invasive method for the measurement of udder tension, suitable for monitoring intra-mammary pressure of an individual dairy cow. At the same time, milk intervals can be tailored to these individual characteristics of the dairy cow to optimize individual milk production.

A method according to the present invention may be used in research as well as in practical applications like milking robots, to objectively determine the tension of the udder of an individual dairy cow. Based on this parameter, management decisions can be taken with regard to the optimal milking interval for the individual dairy cow.

Another application relates to the diagnostic value of udder tension. Infection of the udder (mastitis) is one the most expensive diseases in dairy cows. Clinical mastitis is often accompanied by swelling and hardness of the udder. A method according to the present invention is suitable for detection of mastitis in the dairy cow and can be easily combined with automatic milking equipment.

The availability of tools for assessment of udder tension enables the development of management practices and breeding stategies that safeguard the welfare of the high yielding individual dairy cow, even when the efficiency of cheap and quality milk production needs further improvement.

It will be obvious to the person skilled in the art that a method according to the present invention can similarly be used for other mammals and other parts of animal bodies, for example muscle tissue and the like.

In a preferred embodiment a method for measurement of the tension in an udder or the like of a live mammal according to the present invention is characterized by the features of claim 10.

With such method the milking intervals of individual mammals, for example cows can be easily optimized based on the established relations. These optimal milking intervals can then be used for the control of milking means, thus optimizing the milking results of each individual mammal.

The invention further relates to a measuring device for use in a method according to the present invention, characterized by the features of claim 11.

In a preferred embodiment a measuring device according to the present invention is characterized by the features of claim 16.

A device of a self learning type enables constant adjustment of the system based on the measured relations of the respective tissues and for example secundary, subjective evaluation of the same tissue by a reference panel, which data can be introduced into said device.

Other secundary parameters, which can be for example measured with known methods, and which can be fed into said device can be for example pH, connective tissue, sarcomere length, fat depth, intramuscular fat, colour, texture and the like.

The present invention further relates to the use of a method or measuring device according to the present invention in the field of meat treatment, characterized by the features of claim 21.

Furthermore, the present invention relates to the use of a method or a measuring device according to the present invention in the field of milking mammals, characterized by the features of claim 22. Such method is especially though not exclusively suitable for investigative and diagnostic purposes.

The present invention also relates to a slaughter house, designed for use of a method or provided with a measuring device according to the present invention.

The present invention also relates to a milking installation, especially a milking robot, designed for use of a method or comprising a measuring device according to the present invention.

Further advantageous embodiments of a method, device, use, slaughter house and milking installation according to the present invention are given in the subclaims.

To further clarify the invention, exemplary embodiments of a method and apparatus according to the present invention will be described hereafter with reference to the drawings.
Fig. 1 shows a first embodiment of a measuring device according to the present invention, in partial cross-section;
fig. 2 shows a second embodiment of a measuring device, in partial cross-section;
fig. 3 a third embodiment of a measuring device, in partial cross-section;
fig. 4 a fourth embodiment of a measuring device, in partial cross-section;
fig. 5 a fifth embodiment of a measuring device, in side view;
fig. 6 diagrammatically a number of measurement results of a measuring device according to the present invention;
fig. 7 schematically a slaughter house, provided with measurement devices according to the present invention; and
fig. 8 a milking station according to the present invention.

Throughout the description corresponding parts have corresponding reference signs. The embodiments described are shown as examples and do not limit the scope of the present invention.

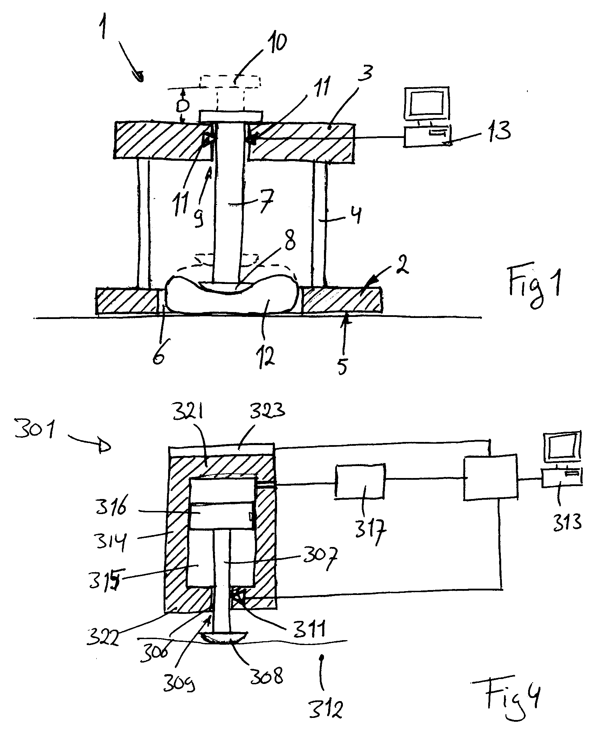

Fig. 1 shows a first embodiment of a measuring device 1 for measurement of tension or tenderness of animal tissue. The device 1 comprises a ring shaped support structure 2 on which a measurement table 3 is carried by support columns 4. The support structure has a flat supporting face 5 facing away from the measurement table 3. The ring shaped support structure 2 circumscribes a central opening 6. Through the center of the measurement table 3 a measurement column 7 extends, carrying a measurement head 8 at the bottom end thereof, which measurement column 7 extends perpendicular to the supporting face and is movably guided in its axial direction by a slide bearing 9 in the measurement table 3. On the top end of the measurement column 7 an end ring 10 is positioned, preventing the measurement column 7 to pass the slide guiding 9. When the measurement column 7 is in its lowest end position, shown in full lines in figure 1, the end ring abuts the top side of the measurement table 3 while the bottom side of the measurement head 8 is positioned at a slight distance from the supporting face 5.

Within the slide guiding 9 reference means 11 are provided for registering the movement of the measurement column 7 relative to the measurement table 3. The reference means 11 are connected to a computer 13 comprising an algorithm to be discussed hereafter.

A measurement device 1 can be used as follows.

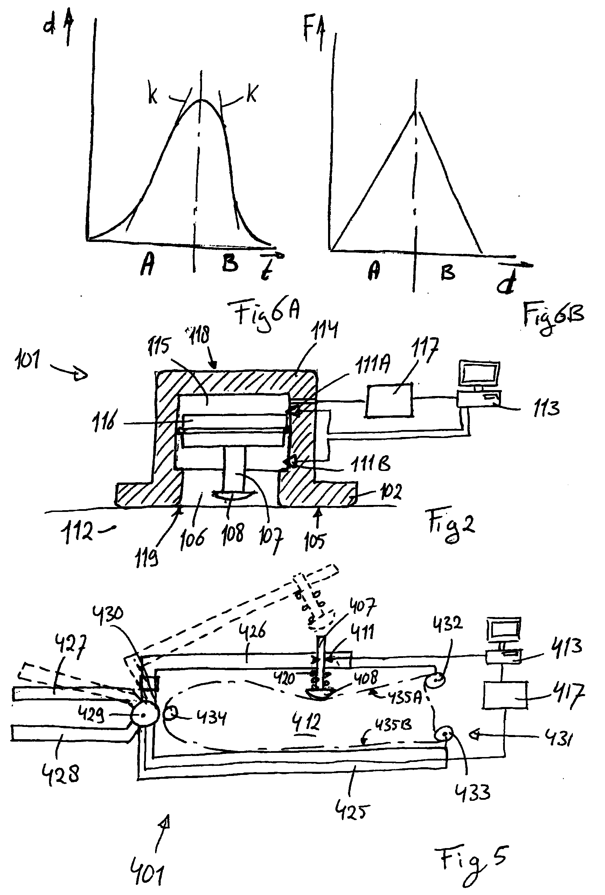

A sample 12 of animal tissue to be measured, for example a piece of meat is positioned within the opening 6 under the measurement head 8, which is thus moved upward over a distance D. The supporting face 5 is firmly positioned on for example a table top. The measurement column 7 is then pushed down over a predetermined distance, for example said distance D, which is registered by the reference means 11 and fed into the computer 13, if desired together with the time necessary for said movement. During the downward movement the measurement head 8 is pushed against the top surface of the sample 12, thus deforming said surface into a dent, as shown in full lines in figure 1. The measurement column 7 is then released, such that it can then be pushed upward by the resilience of the sample 12. During this upward movement the distance and time required are registered by the reference means 11 and also fed into the computer 13. The distance D and time T measured can be plotted in a diagram as shown in figure 6a and can be stored in the computer memory. In said memory, reference data are stored of samples having known properties such as tension, tenderness, origin and the like and preferably data about the appreciation of said samples by a reference panel. The data obtained with the measurement device 1 of said shown sample 12 can then be compared to the set stored in the computer 13, from which comparison the tension or tenderness of the sample 12 can be assessed by means of said algorithm. The data about specific samples can be added or substracted from the set stored in the computer in order to keep the reference set up to date and accurate.

The complete diagram as shown in figure 6a can be used for the comparison as described here above, but in a preferred embodiment only part of the data obtained during the upward movement of the measurement column 7 and measurement head 8 is being used, since said upward movement is exclusively caused by the resilience of the sample and is uninfluenced by the operator. Therefore the data are not biased by the way the operator moves the measurement column 7, for example with respect to speed, acceleration, force and direction. Therefore objective data are obtained. In a further preferred embodiment only the data obtained in the middle part of the upward path of travel of the measurement column is being used, indicated in figure 6a by the line K, since then disturbances due to the beginning and the end of movement are excluded from said comparison.

Figure 2 shows a second embodiment of a measurement device 101 according to the present invention, which can be hand held. The measurement device 101 comprises a housing 114, provided with a ring shaped support structure 102, circumscribing an opening 106. Within the housing 114 a cilindrical chamber 115 is provided in which a piston 116 is movable in a direction perpendicular to the supporting face 105 of the support structure 102. Connected to the piston 116 is a measurement column 107 carrying a measurement head 108 at its free end, which measurement head 108 is movable through the central opening by the piston 116. When the piston 116 is moved fully upward, the measurement head extends in the opening 106 within said housing 114. When the measurement head is moved downward the measurement head 108 extends outside the housing over a distance D, as shown in dotted lines in figure 2.

The chamber 115 is connected to pressure means 117, such as a pump, for introducing a pressure fluid into said chamber 115, thus forcing the measurement head 108 outward. The pressure means 117 are designed to be able to also retract the pressure fluid from said chamber 115, thus moving the piston 116 and measurement head 108 back into said housing. Within the wall of the housing 114 a top reference means 111A and a bottom reference means 111B are positioned above each other for measurement of the movement of the piston 116 relative to the housing 114. The reference means 111A,B are connected to a computer 113, comprising an algorithm as discussed hereabove.

A measurement device 101 according to figure 2 can be used as follows.

The housing 114 is held in the hand of an operator, the top side 118 thereof positioned against the palm of said hand. The support face 105 is then pressed against the top surface of the sample 112, the head 108 being retracted. The support face 105 is in abutment with the sample 112 without deformation. Then pressure is applied to the piston 116 by means of the pressure means 117, thus forcing the measurement head 108 against the top surface of the sample 112, thereby deforming the sample 112. The path of travel of the measurement head 108 is measured by the reference means 111A,B and fed into the computer while the pressure, necessary for said movement is also measured and fed into said computer 113. If appropriate, the path of travel and the pressure exerted by the piston on the pressure fluid during an upward stroke of the measurement head 108, due to the resilience of the sample, can be measured as well, which would result in a diagram as for example shown in figure 6B, A representing the force during the downward stroke, B during the upward stroke.

A device 101 according to figure 2 has the advantage that it can be hand held and can be very easily operated in sito. Since all movements of the measurement head are caused hydraulically and/or mechanically, chances of bias due to the manner of operation by the operator are excluded. The path of travel and the distance between the inside edge 119 of the support structure and the axis of the measurement column 107 can be easily chosen such that the deformation of the sample is constricted mainly to the area covered by the opening, thus further preventing bias by parasitic movement of the housing 114, though such problem is mainly avoided since movement of the reference head is measured relative to said support structure. It should be noted that a device according to figure 2 can also be brought in abutment with a sample by mechanical means, for example by using a robotic arm or the like.

In operating a device 101 according to figure 2 it is also possible to move the piston over a preset distance, for example between the reference means 111A and 111B, whereby the pressure applied by the pressure means 117 is monitored and used for comparison purposes. It is furthermore possible to use a preset pressure by the pressure means 117, whereby the path of travel of the piston in the downstroke and/or upstroke is measured and used for comparison purposes. Moreover, acceleration and/or speed can be measured instead of distance and time as can change in speed or acceleration or a combination thereof. These can be related to for example time or pressure data. A method preferred depends mainly on the resilience of the sample, the possible path of travel and the algorithm to be used. The supporting face 105 is preferably relatively large compared to the size of the measurement head, so that the head 108 can deform the surface of the sample 112 relatively easy whereby the pressure applied to the housing is evenly distributed over the supporting face 105, thus preventing deformation by the housing.

Figure 3 shows a third embodiment of a measurement device 201 according to the present invention. In this embodiment the device 201 comprises a housing 214, enclosing a chamber 215, connected to the outside by an opening 206 in the bottom part of the housing 214. Through the opening 206 a measurement column 207 extends, guided by slide guiding 209. On the top end of the measurement column an end ring 210 is provided, which cannot pass the opening 206. A measurement head 208 is provided at the opposite end of the measurement column 207. Within the chamber 215 a spring member 220 is provided between the top side of the end ring 210 and the inside of the top wall 221 of the housing 214. The end ring 210 and thus the measurement column 207 and measurement head 208 are biased into the position as shown in figure 3 by the spring member 220, whereby the end ring abuts the inside of the bottom wall 220 of the housing 214. The end ring 210 and the column 207 and head 208 can be moved upward relative to the housing 214 against the resilience of the spring member 220. Within the slide guiding 209 first reference means 211 are provided for measurement of the movement of the measurement column 207 relative to the housing 214, which first reference means 211 are connected to a computer 213. Against the outside wall of the housing 214 a second reference means 223 is provided, comprising means for measuring the distance between said second reference means 223 and a surface 212a of the sample 212 against which the measurement head 208 is forced as will be explained hereafter. The second reference means 223 can for example comprise laser operated distance measurement means as are known by the skilled person, using the reflection of a laser beam on the said surface 212a. Other conventional distance measurements means may be used, such as a rod touching said surface 212a, which can be moved up and down relative to said housing 214 while in contact with said surface without deforming the latter. The second reference means 223 are also connected to the computer 213.

A device according to figure 3 can be used as follows.

The housing 214 is held by an operator, the measurement head brought into contact with the surface 212a of the sample 212. The housing 214 is then pushed into the direction of the sample 212, against the resilience of the spring member 220. The distance over which the end ring 210 is moved into the chamber 215 is measured with the first reference means, whereby the movement of the housing relative to the non-deformed part of the surface 212a is measured with the second reference means 223. The difference between the said distances D1 and D2 is then established by the algorithm in the computer 213 and compared with a reference set stored in said computer, as described hereabove.

A measurement device 201 according to figure 3 has the advantage that only the measurement head 208 has to be brought into contact with the surface 212a of the sample, thus avoiding deflexion of said surface by a support structure as shown in figures 1 and 2.

In the method described hereabove in using the measurement device 201 the surface 212a of the sample 212 is being used as a reference surface for the second reference means 223. However, it is also possible to use an external surface 212B supporting the sample to be measured, as is shown in figure 3A. Such method has the advantage that the measurement obtained by the second reference means 223 is not influenced by deformations of the sample surface 212A. By providing second reference means 223 at opposite sides of the housing 214 or, preferably, three sets of second reference means 223 evenly spaced around said housing 214 the algorithm in said computer 213 can compensate for any deviation in the direction of movement of the device from perpendicular to said supporting surface 212B.

Figure 4 shows a fourth embodiment 301 according to the present invention, comprising a housing 314 enclosing a chamber 315, an opening 306 extending through the bottom wall 322 of the housing 314. Within the chamber 315 a piston 316 is positioned, connected to a measurement column 307 extending through said opening 306 and a slide guiding 309 positioned therein. At the free end of the measurement column 307, outside said housing 314 a measurement head 308 is provided. Pressure means 317 are connected to the chamber 315 above the piston 316 for introducing or retracting pressure fluid into or from said space above the piston for forcing the piston 316 into the direction of the bottom wall 322 of the housing 314 or in the opposite direction, thus moving the measurement head 308. Reference means 311 are provided in the slide guiding 309 for measurement of the movement of the measurement column 307 relative to the housing 314. On the top wall 221 of the housing 314 second reference means 323 are provided in the form of pressure sensing means. The pressure means 317, the first reference means 311 and the second reference means 323 are all connected to a computer 313 comprising an algorithm as discussed hereabove.

A measurement device 301 according to figure 4 can be used as follows.

The piston 316 is moved into a middle position within the chamber 315 or at least to a distance spaced apart from the top wall 321 of said chamber 315. The reference head 308 is then forced against the top surface of a sample 312, whereby a hand of the operator is positioned on top of the second reference means 323. The housing 314 is then forced into the direction of the sample 312, whereby the pressure applied to the second reference means and the path of travel of the housing relative to the measurement column 307 is recorded by the first reference means 311. During this movement the pressure applied by the pressure means 317 and the piston 316 relative to each other is monitored and preferably kept constant. Thereby a pressure/path of travel ratio is obtained which can be compared by the algorithm to a reference set stored in said computer 313. Obviously it is also possible to variate the pressure applied to the piston by the pressure means 317, for example to provide for a larger or smaller path of travel or for keeping the pressure applied to the second reference means 323 at a predetermined, constant or variating level. The device according to figure 4 can be hand held or mechanically operated, for example by a robot arm.

Figure 5 shows a fifth embodiment of a measuring device 401 according to the present invention. This device 401 is tong shaped, having a first tong jaw 425 and a second tong jaw 426, operated by pressurizing a first lever 427 and a second lever 428. A closing pressure can be applied by hydraulic means 429, connected to pressure means 417 or adjustable spring means like in gripping tongs, for applying a predetermined closing pressure and -speed, from the open position, shown in broken lines in figure 5, to the closed position, shown in solid lines. The first tong jaw 425 is relatively long and wide and forms a support surface for a sample to be measured. From the second tong jaw 426, which can be relatively short and narrow compared to the first tong jaw 425, a measurement column 407 extends, which is movable in its axial direction, perpendicular to the surface of the first tong jaw 425 when the tong is in the closed position. The measurement column 407 is provided with a measurement head 408 at the end facing the first tong jaw 425 and is biased in the direction of the first tong jaw by a spring member 420. First reference means are provided for measurement of the movement of the measurement column 407 relative to the second tong jaw 426, which first reference means are connected to a computer 413 having an algorithm as described hereabove. The pressure means 417 are also connected to said computer 413, thus providing data about the applied pressure, closing speed and/or closing pressure. Preferably adjustment means 430 are provided in at least one of the tong jaws 425, 426, for adjustment of the distance between the said tong jaws. The initial distance between the top surface of the first tong jaw 425 and the measurement head 408 can thus be adjusted to the thickness of the sample to be measured. The measurement head can be provided with a load cell, connected to the computer 413, for measurement of the pressure applied and/or determining the moment of contact between the measurement head 408 and the sample for compensating for differences in sample thickness.

The first and second reference means provided for in a measurement device according to the present invention, as shown in the figures 1-5 can be chosen according to the data to be measured. Ordinary transponders can be used for measurement of displacement, speed, acceleration and/or changes therein or transonders for measurement of pressure or change in pressure or any combination thereof. These can be of any known contact or non-contact type, such as optical means, electro-mechanical means, strain gauges and the like. The appropriate choice will be sufficiently clear to a person skilled in the art.

In the embodiments shown the first reference means 11-411 are all provided near the measurement column 7-407. However, it will be clear that these first reference means can be placed in a different position, for example for measurement relative to the end ring or piston as shown in figs. 1, 3 and 2, 4 respectively. In the embodiment as shown in figure 5 the angle between the first and second tong jaws 425, 426 can also be measured and fed into the algorithm within said computer 413.

In each of the embodiments shown further measurement means can be provided for measuring secondary parameters, such as pH connective tissue, sarcomere length, fat depth, intramuscular fat, colour, texture and the like, the data of which can also be fed into the computer. For use in said algorithm for establishing the tension or tenderness of the sample. Such means are known to a person skilled in the art and for example described in the book "On-line evaluation of meat" (H.J. Swatland; published by Technomic Publishing Company, Lancaster, Basel; 1995) incorporated herein by reference. A measuring device according to the present invention can be used in for example a butchery or a milking station, to be described hereafter, or in breeding and selection procedures for live stock, based on for example muscle growth, meat quality and fat, as for example described in "Selection on intramuscular fat in Swiss pig breeds and the importance of fatty tissue quality", Schwörer, D. et al; second Dummerstorf Mucle-Workshop Muscle Growth and Meat Quality; Rostock, 17-19^{th} May 1995, Proceedings; p. 116-124, incorporated herein by reference.

The measurement head 8-408 preferably has a smooth surface, curved into the direction of the sample, and has a relatively small surface compared to the surface of the sample. Therefore the deformation of the surface is local while damage of the surface by the measurement head is avoided. A method according to the present invention is thus primarily non-invasive as far as the measurement head is concerned.

Figure 5 shows schematically means for covering the measurement head 408 and the first tong arm 425 and second tong arm 426 during measurement of a sample. The covering means 431 comprises a first 432 and second carrier 433 near the free ends of the respective tong arms 426 and 425. The first carrier 432 carries a row of foil, which can be rolled up on the second carrier 433. Near the rotating point 429 a third carrier 434 is provided over which the foil 435 is led between the first 432 and second carrier 433. During measurement the sample is positioned between the two layers of foil 435A and 435B, thus preventing contamination of the sample by the tongs and vice versa. When a new sample is entered between the tong arms the foil 435 is rolled up, such that the whole contact area between the tongs and the sample is covered by a new sheet of foil 435a,b. This is preferably carried out automatically. It is also possible to dispense of the third carrier, the foil being pushed into the direction of the rotating point 429 by the sample introduced and automatically rolled up by the second carrier 433 when the sample is removed. Thus the hygiene of the device is further enhanced. Comparable foil covering means can be provided with any of the other embodiments of a device according to the present invention.

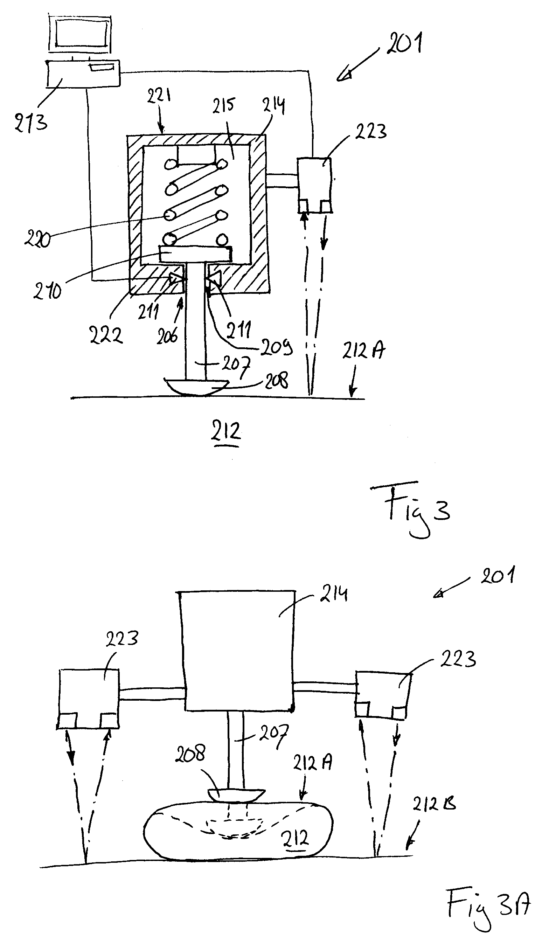

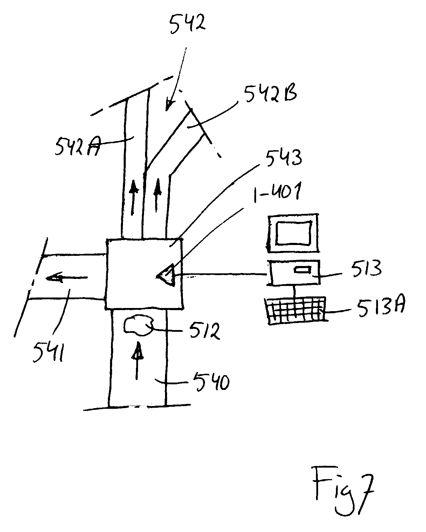

Figure 7 schematically shows part of a butchery 500 according to the present invention, comprising of a first transporting means 540, second transporting means 541 and third transporting means 542, for example conveyor systems comprising hook members for hanging meat, conveyor belts or the like. The first transporting means 540 transports meat to a selection station 543 in which at least one measuring device according to the present invention is provided, in a suitable embodiment 1-401. Meat is transported into the selection station 543 by the first transporting means 540, in which selection station the relevant data are obtained by means of the measurement device 1-401, which data are fed into the computer 513. The tension or tenderness and possibly further characteristics of the relevant piece of meat is assessed by the algorithm in the computer 513, compared to the relevant data set present in said computer 513. The computer 513 then controls the selection station for further processing of said relevant piece of meat. When the piece of meat does not stand up to the standard set it will be dispelled from the selection station 543 by means of the second transporting means 541, for example for disposal or use in animal Feed production or the like. If the meat is approved of it can be transported from the selection station 543 by the third transporting means 542, for example by the left conveyor belt 542A if it is "first class" meat or by the right conveyor belt 542B if it is "second class" meat. Thus an automatic selection of the meat can be provided for. At specific intervals a sample can be taken out and can be assessed by for example a human panel, the data obtained from said panel being entered into the computer 513 by means of a keyboard 513A or the like. The selection criteria for the selection station 543 can then be adjusted by said panel data, if necessary.

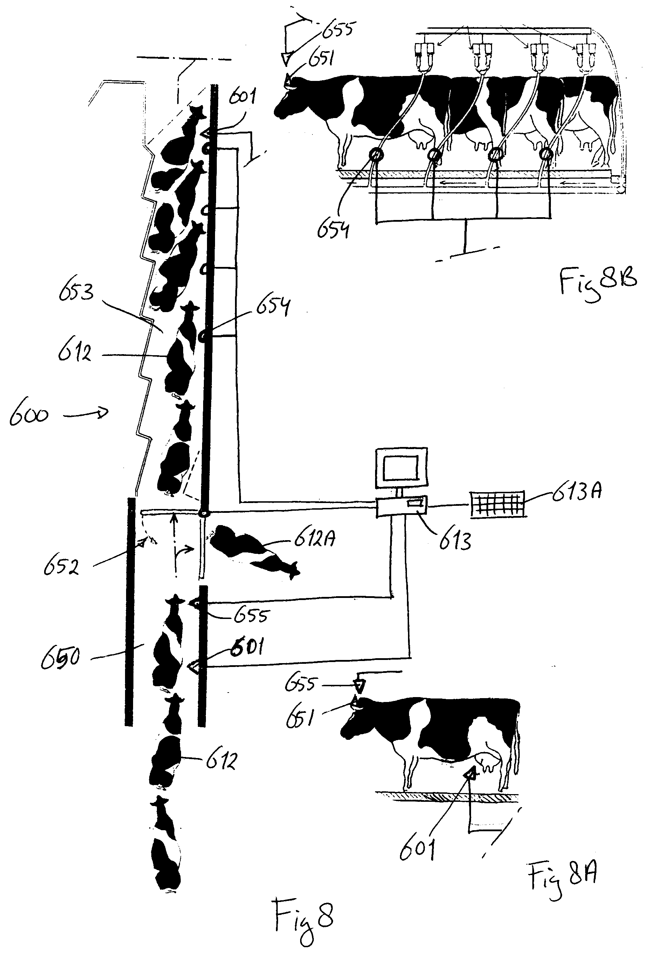

Figure 8 shows schematically in top view a milking station 600 in which milking cows 612 can be lead automatically. The cows 612 enter an entrance corridor 650, carrying a transponder 651, for example in their ear as shown in figure 8A. The individual cow 612 can then be recognized by the computer based on the response from said transponder through a responder 655. Furthermore, in or near the entrance corridor 650 at least one measuring device 601 according to the present invention can be positioned at will, for measurement of the tension of the udder of said individual cow 612, preferably at specifically chosen intervals. The measured tension of the udder is fed into the computer 613 and compared to the relevant data of said individual cow, for assessment of the amount of milk stored in said udder. If the udder seems sufficiently filled a control gate 652 is brought into a first position, connecting the entrance corridor 650 with the milking area 653. The individual cow 612 is then lead into a milking position as shown in the top half of figure 8 and in figure 8b. During milking of the cow the yield of the individual cow is measured by third reference means 654, the data of which are fed into the computer 613. The algorithm within said computer 613 then provides relevant data about the relation between the tension in the udder as measured upon entrance of the entrance corridor and the actual yield during milking. The algorithm then provides for an optimum milking interval for said individual cow in order to obtain a maximum yield. If desired, a second measuring device 601 according to the present invention can be used within or behind the milking station for assessment of the tension in an empty udder, for control purposes. When a cow 612 enters the entrance corridor 650 with a (probably) partly empty udder, this will be detected by the measuring device and/or the computer, which will then close the milking corridor 653 by means of the control gate 652, thus directing the individual cow 612A back into the field or stable for further grazing. With a milking station according to the present invention it is thus possible to automatically set optimal milking intervals for each individual cow in order to obtain maximum yield. The same or a similar algorithm can be used with other means for measuring the tension in the udder and/or means for providing relevant data about the filling of the udder and the yield of the relevant cow.

When the data of the cows 612 is stored in the computer the determined milking interval can also be used for operating the control gate, the measuring device 601 being used for periodically checking the relevant data.

A further advantage of a milking station according to the present invention is that each individual cow can be checked for a number of diseases, for example mastitis, which can be detected by tensioning of the udder. Since each cow will pass the milking station a number of times each day the occurance of said diseases can be detected at a very early stage, thus preventing decline of the health of said cows.

The invention is not in any way limited to the embodiments shown or described in this publication, many variations are possible within the scope of the present invention.

Any combination of at least time, displacement and/or pressure measured can be used with a method or device according to the present invention for comparison purposes. Two or more measurement heads can be provided for even more accurate measurement on different parts of a sample. A method and apparatus according to the present invention can be used with other animals, such as sheep, goats and the like and can be used for the detection of different diseases resulting in tensioning of tissue. It will be directly apparent that a device and method according to the present invention can be used with live stock as well as dead meat. Different methods for displacement of the measurement head can be used, in any necessary direction.

These and comparable variations are understood to fall within the scope of the invention.

## Claims

1. Method for measurement of tension or tenderness of animal tissue such as meat, using an instrument having at least one measurement means and reference means, whereby the measurement means is positioned in a first position, against part of a tissue to be measured, whereafter the measurement means and/or the reference means are moved relative to the tissue to a second position, thereby deflecting the tissue, after which the measurement means and/or the reference means are moved back in the direction of the first position, wherein during at least part of the path of travel of the measurement means the relation is established between at least two of the following parameters of the movement of the measurement means relative to the reference means:
time, displacement, speed, change in speed, acceleration,
change in acceleration, duration of movement, pressure,
change in pressure,
which relation is used for assessment of said tension or tenderness.

2. A method according to claim 1, wherein said relation is compared to a standard set of relations for similar tissue having known, specific tension or tenderness, from which comparison the tension or tenderness of the measured tissue is determined.

3. A method according to claim 1 or 2, wherein said parameters are measured at least during movement of the measurement means from the first position in the direction of the second position.

4. A method according to any one of the preceding claims, wherein said parameters are measured at least during movement of the measurement means from the second position in the direction of the first position.

5. A method according to any one of claims 1 - 4, wherein the measurement means is moved from the first position to the second position by mechanical means.

6. A method according to any one of claims 1 - 5, wherein the measurement means is moved against the surface of said tissue and does not penetrate said tissue.

7. A method for measurement of tenderness of meat in a slaughterhouse, meat industry, butchery or the like, wherein meat is transported relative to a measuring device for performing a method according to any one of the preceding claims, wherein the measurement means is moved against the meat and the necessary parameters are measured, after which said relation is established and compared with said relevant reference set, after which the meat is treated further based on said relation.

8. A method according to claim 7, wherein electronic, means of a self learning type are use for comparison of said relation with said reference set, wherein at intervals the tenderness or tension of said measured meat is evaluated by human interference, said evaluation entered into said electronic means, said electronic means comprising an algorithm for amending said reference set based on said assesment of the tenderness or tension and said relevant evaluation.

9. A method for measurement of the tension in an udder or the like of a live mammal, wherein a method according to any one of the claims 1 - 6 is used.

10. A method according to claim 9, wherein a milking interval for an individual mammal is established, whereby electronic means of a self learning type are use for comparison of said relation with said reference set, wherein the tension of said measured udder is related to the filling of said udder, said relation entered into said electronic means, said electronic means comprising an algorithm for amending said reference set based on the tension of the udder and the filling and establishing the milking interval of the relevant mammal.

11. Measuring device for use in a method according to any one of the preceding claims, comprising a measuring means movable relative to reference means, wherein means are provided to register at least two of the following parameters of the movement of said measuring means relative to said reference means:
time, displacement, speed, change in speed, acceleration,
change in acceleration, duration of movement, pressure,
change in pressure,
wherein comparison means are provided for defining a relation between at least two of said measured parameters and communication means for communicating said relation.

12. Measuring device according to claim 11, wherein actuating means are provided for controlled movement of said measuring means in at least one direction.

13. Measuring device according to claim 11 or 12, wherein said communication means comprises display means.

14. Measuring device according to any one of claims 11 - 13, wherein comparison means are provided for comparison of the or each relation to a reference set of relations of tissues with known tension or tenderness and defining the tension or tenderness of the measured tissue based on said comparison.

15. Measuring device according to claim 14, wherein control means are provided for control of machinery for for example treatment of said tissue or tissue related animals, based on said definition of the tension or tenderness of the measured tissue.

16. Measuring device according to any one of claims 11 - 15, wherein the device is of a self learning type, comprising:
- a reference set of relations for similar tissue having known, specific tension or tenderness;
- means for comparing measured parameters with said reference set;
- means for determining the tension or tenderness of the measured tissue from said comparison;
- means for reading external parameters into said device;
- an algorithm for adjustment of said reference set and/or said comparison based on said measured parameters and said external parameters.

17. Measuring device according to any one of claims 11 - 16, wherein second measuring means are provided for measurement of at least one of the parameters:
pH, connective tissue, sarcomere length, fat depth, intramuscular fat, colour,
whereby means are provided for combination of this at least one parameter with the parameters measured with said first measurement means.

18. Measuring device according to claims 16 and 17, wherein said second measurement means are designed for measurement of at least a number of said external parameters, whereby said second measurement means comprises at least part of said means for reading the external parameters into the device.

19. Measuring device according to any one of claims 11 - 18, wherein the measuring means comprises at least one measuring head and at least one reference head, means for registering said parameters being provided on the measuring head and/or the corresponding reference head, the or each reference head being provided with a reference plane at least partly surrounding the or each corresponding reference head and designed to abut the meat or muscle or the like to be measured in a non-invasive manner.

20. Measuring device according to any one of claims 11 - 19, wherein the or each measuring means is designed to provide during use at least mainly electronical signals equivalent to the parameters measured.

21. Use of a method according of any one of claims 1 - 10 or a measuring device according to any one of the claims 11 - 20 in the field of meat treatment, especially for determining the optimal path of treatment of meat in a butchery or the like, wherein apparatuses are operated at least partly based on signals from said measuring device.

22. Use of a method according of any one of claims 1 - 10 or a measuring device according to any one of the claims 11 - 20 in the field of milking mammals, especially for determining optimal milking intervals or for determining mastitis and like diseases.

23. Slaughter house, designed for use of a method according to any one of claims 1 - 10 or comprising at least one measuring device according to any one of claims 11 - 20.

24. Milking installation, especially a milking robot, designed for use of a method according to any one of claims 1 - 10 or comprising a measuring device according to any one of claims 11 - 20.
